# EUROPEAN PATENT SPECIFICATION

(11) **EP 0 855 179 B1**
(45) Date of publication and mention of the grant of the patent: **13.11.2002**
(21) Application number: 97500231.2
(22) Date of filing: 31.12.1997
(51) Int. Cl.: A61K 9/127, A61K 9/48, A61K 9/30

(54) **Pharmaceutical preparation comprising coated capsules or tablets containing a liposome powder encapsulating a drug**
Pharmazeutische Zubereitung umfassend mit einem Überzug versehene Kapseln oder Tabletten enthaltende in einen Wirkstoff enthaltendes Liposomenpulver
Préparation pharmaceutique comprenant des capsules ou des comprimés contenant des liposomes sous forme d'une poudre qui renferment un ingrédient actif

(30) Priority: 16.01.1997 ES 9700073
(43) Date of publication of application: 29.07.1998
(73) Proprietor: Lipotec, S.A., 08902 L'Hospitalet de Llobregat (Barcelona) (ES)
(72) Inventor: Garces Garces, Josep, 08620 Sant Vicen dels Horts, Barcelona (ES); Bonilla Munoz, Angel, 08960 San Boi de Llobregat, Barcelona (ES); Parente Duena, Antonio, 08960 Sant Just Desvern, Barcelona (ES)
(74) Representative: Davila Baz, Angel

(56) References cited:
- EP-A- 0 366 277
- WO-A-97/27843
- ES-A- 2 034 877
- FR-A- 2 581 543
- DATABASE WPI Section Ch, Week 8918 Derwent Publications Ltd., London, GB; Class B03, AN 89-132542 XP002091456 & JP 01 075421 A (SAWAI SEIYAKU KK) , 22 March 1989
- DATABASE WPI Section Ch, Week 8528 Derwent Publications Ltd., London, GB; Class A96, AN 85-168491 XP002091457 & JP 60 097917 A (TERUMO CORP) , 31 May 1985

## Description

### Scope of the invention

This invention relates to a new composition for improving the absorption of orally administered drugs.

The composition according to the invention is obtained by incorporating the drug in liposomes comprising hydrogenated lecithin and cholesterol, followed by evaporation or freeze-drying of the solvent in which the liposomes are suspended.

The resulting liposome powder is administered orally, in a capsule or tablet, protected in its passage through the stomach by an enteric coating.

### Basis of the invention

The oral route is the preferred route for the administration of drugs of all kinds to patients because of the advantages offered by this type of administration in comparison with other more aggressive routes and/or routes which are of more difficult application (endovenous, parenteral, subcutaneous, etc.).

Nevertheless, not all drugs are satisfactorily absorbed via the gastrointestinal tract. This absorption depends, among other factors, on the permeability of the gastrointestinal mucosa to the drug and on the acid and/or enzyme degradation processes to which the drug is subjected during its residence time in the gastrointestinal tract.

It is therefore obvious that any factor which improves the rate of absorption of the drug or which protects it from the degradative processes mentioned will improve the clinical efficacy of the drug.

### State of the Art

Great efforts have been made recently to identify agents which are capable of increasing the permeability of the gastrointestinal mucosa to poorly absorbed materials: surfactants (George, Sutter, Finegold, J. Infect. Dis. 136, 822 (1977)), chelating agents (Cassidy, Tidball, J. Cell. Biol. 32, 672 (1967)), salicylates (Higuchi, et al., U.S. Patent 4,462,991 (1984)), anti-inflammatories (Yaginuma, et al., Chem. Pharm. Bull. 29, 1974 (1961)), phenothiazines (Alexander and Fix, U.S. Patent 4,425,337 (1984)), acyl carnitines (Alexander and Fix, USSN 606,054), and fatty acids (Yamazaki, et al., J. Pharm. Pharmacol., 42, 441, (1990)) have been described as being capable of increasing the gastrointestinal permeability of a great variety of compounds. On the other hand, the effort to produce systems which protect a drug from gastrointestinal degradative processes has also been very considerable. Amylose coatings (WO 89/11269), lactic and glycolic acid polymers (EP 0202159), calcium alginate (Chong-Kook K., Eun-Jin L., Int. J. Pharm., 79, 11 (1992)), and polymerized liposomes (WO 95/03035) have been described as systems for the administration of drugs via the gastrointestinal route.

### Summary of the Invention

As a result of the research work carried out it has now been surprisingly found that the bioavailability of drugs which are difficult to absorb via the gastrointestinal route is greatly increased when they are administered orally incorporated in freeze-dried liposomes, provided that this freeze-dried liposome preparation is administered in capsules or tablets provided with an enteric coating which enables them to withstand the acid environment of the stomach.

The subject matter of this invention therefore comprises incorporating a drug in liposomes comprising hydrogenated lecithins and cholesterol, subsequently freeze-dried or evaporated to dryness to obtain a powder, which can be used as a new system for the oral administration of drugs in tablets or capsules provided with a coating of enteric material which makes it possible to release the contents of these capsules or tablets after they have passed through the stomach.

These formulations, when administered orally, have the inherent advantages in comparison with other kinds of administration, that is, these formulations are easier to administer, more convenient, less aggressive and better tolerated by patients.

### Detailed description of the invention

Lecithins are mixtures of phospholipids obtained by extraction from natural products such as eggs (egg lecithins), soya (soya lecithins), etc. These natural lecithins are in fact mixtures of phospholipids comprising fatty acids of different chain length and different degrees of saturation.

In this invention hydrogenated lecithins are taken to mean lecithins obtained from natural sources (such as eggs, soya, etc.) which have been subjected to a catalytic hydrogenation process with a view to reducing their degree of unsaturation to a very low level (iodine number not greater than 2 g/100 g).

The constituent lipids of the liposomes comprising the system according to this invention comprise a mixture of hydrogenated lecithins and cholesterol in which the proportion of cholesterol varies between 0 and 75%, and more specifically between 24% and 51%.

The processes for obtaining liposomes and incorporating the drugs in these liposomes, which is required in order to obtain the system according to this invention, are well-known and described in the state of the art (see, e.g.: Szoka F. and Papahadjopoulos, D., Ann. Rev. Biophys. Bioeng. 9 (1980) 467-508).

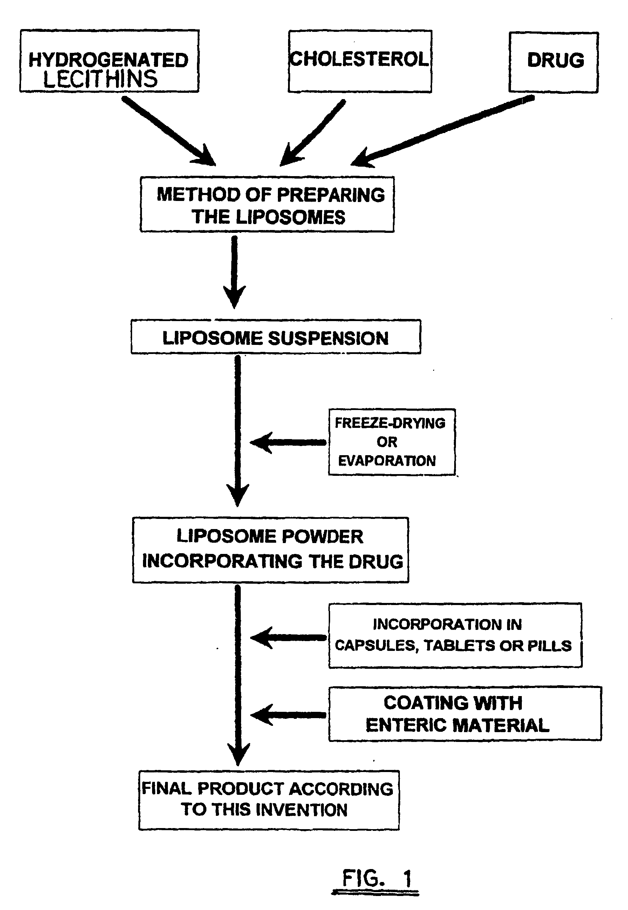

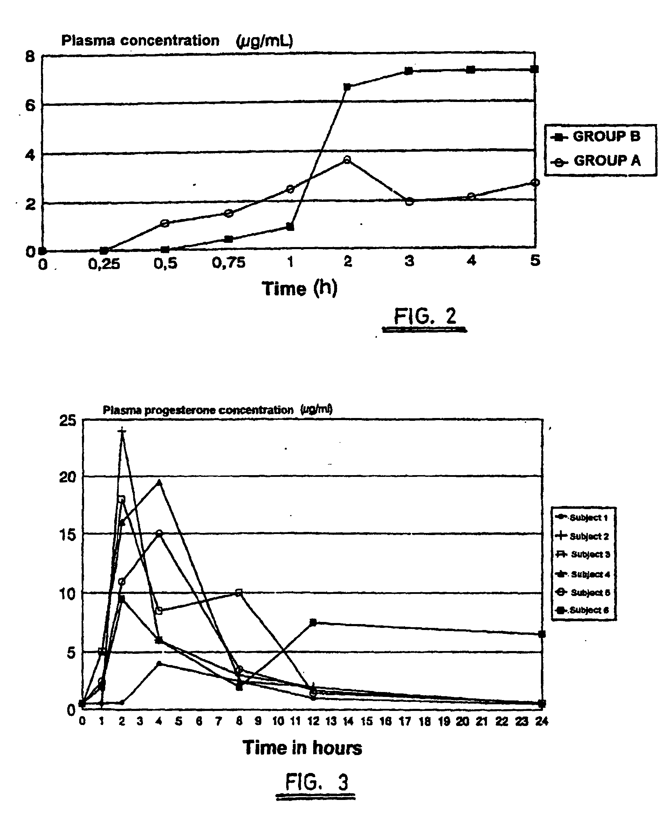

Appended Figure 1 shows a flow diagram of the process for obtaining the composition according to this invention.

The materials which can be used to manufacture the enteric coating for the capsules or tablets to which this invention relates are widely known in the state of the art and are commonly used in the pharmaceutical field to produce enteric coatings for capsules and tablets, such as, e.g. seals based on acrylic resins (acrylic acid copolymers with acrylic acid esters), e.g. Eudragit L™, Eudragit S™, Eudragit RL™ or Eudragit RS™, etc., and seals based on cellulose derivatives such as cellulose acetophthalate, etc.

The drugs which can be incorporated in the freeze-dried or evaporated liposome powder, which further can be incorporated into capsules, tablets or dragees coated with enteric material, according to the invention, comprise, but are not restricted to:
- diphosphonates such as alendronic acid and their salts,
- mineral salts having pharmacological activity, such as iron (II) sulfate, sodium fluoride, etc.

The invention will now be described purely by way of illustration by means of the following examples.

### EXAMPLE 1: PREPARATION OF FREEZE-DRIED LIPOSOME CAPSULES CONTAINING SODIUM ALENDRONATE

A mixture comprising 1244.2 mg of hydrogenated egg lecithin and 499.7 mg of cholesterol was dissolved in 200 ml of chloroform/methanol (75/25). The organic solvents were then removed by rotary evaporation, and traces of solvent were also removed by the passage of N₂ or by freeze-drying. The lipid film obtained in this way was hydrated using 50 ml of 0.9% NaCl solution containing 264.1 mg of sodium alendronate, by agitation in a vibromixer at intervals of 30 seconds, followed by standing in a bath kept at 60°C for the same length of time until the total effective agitation time was 10 minutes, a liposome suspension being obtained in this way.

The resulting liposome suspension was frozen and freeze-dried to obtain a freeze-dried powder which was placed in hard gelatin capsules (50 mg of freeze-dried preparation in each gelatin capsule). The resulting capsules were coated with an enteric polymer (EUDRAGIT-L) by repeated immersion in a solution of the enteric polymer in isopropanol and subsequent drying in a current of air.

### EXAMPLE 2: PREPARATION OF LIPOSOME CAPSULES CONTAINING IRON (II) Sulfate

2.4 g of FeSO₄.17H₂O, 31 mg of ascorbic acid and 31 mg of cysteine were suspended in 100 ml of distilled water. A mixture comprising 800 mg of cholesterol, 800 mg of hydrogenated lecithin and 31 mg of vitamin E acetate was added slowly to the resulting suspension with vigorous agitation. The resulting suspension was heated to 60°C and was kept under agitation under a nitrogen atmosphere until the lipids were totally hydrated, a suspension of liposomes incorporating iron sulfate being obtained.

The resulting liposome suspension was evaporated to dryness to obtain a solid which was placed in hard gelatin capsules (510 mg of solid in each gelatin capsule). The resulting capsules were coated with an enteric polymer (EUDRAGIT-L) by repeated immersion in a solution of the enteric polymer in isopropanol, and subsequent drying in a current of air.

## Claims

1. A pharmaceutical preparation for oral administration which comprises capsules, tablets or dragees coated with enteric material containing a freeze-dried or evaporated liposome powder, not suspended in oil, incorporating diphosphonates such as alendronic acid and its salts.

2. A pharmaceutical preparation for oral administration which comprises capsules, tablets or dragees coated with enteric material containing a freeze-dried or evaporated liposome powder, not suspended in oil, incorporating mineral salts of iron (II) such as iron (II) sulphate.

3. The pharmaceutical preparation as claimed in any of claims 1 and 2, wherein the lipids used in obtaining these liposomes comprise a mixture of cholesterol and hydrogenated natural lecthins (preferably hydrogenated egg lecitin and hydrogenated soya lecithin and their mixtures) having an iodine number not greater than 2 g/100 g.

4. The pharmaceutical preparation as claimed in any of claims 1, 2 and 3, wherein the enteric agents used are those normally used in the pharmaceutical field to produce enteric coatings for capsules and tablets, preferably seals based on acrylic resins (acrylic acid copolymers with acrylic acid esters) such as Eudragit L™, Eudragit S™, Eudragit RL™ o Eudragit RS™, and seals based on cellulose derivatives such as cellulose acetophtalate.

5. The pharmaceutical preparation as claimed in any of claims 1, 2, 3 and 4, wherein the lipids used to obtain these liposomes comprise a mixture of cholesterol and hydrogenated natural lecithins in which the proportion of cholesterol varies between 0 and 75%, and more specifically between 24% and 51%.

## Patentansprüche

1. Pharmazeutische Zubereitung zur oralen Verabreichung umfassend Kapseln, Tabletten oder Dragees, die mit einer enterischen Substanz beschichtet sind und die ein gefriergetrocknetes oder eingedampftes, nicht in Öl suspendiertes Pulver aus Liposomen enthalten, das mit Diphosphonaten wie Alendronsäure und deren Salzen vermengt ist.

2. Pharmazeutische Zubereitung zur oralen Verabreichung umfassend Kapseln, Tabletten oder Dragees, die mit einer enterischen Substanz beschichtet sind und die ein gefriergetrocknetes oder eingedampftes, nicht in Öl suspendiertes Pulver aus Liposomen enthalten, das mit mineralischen Salzen des Eisen (II) wie Eisen-II-sulfat vermengt ist.

3. Pharmazeutische Zubereitung nach einem der Ansprüche 1 oder 2, wobei die zum Erhalt der Liposome verwendeten Lipide eine Mischung aus Cholesterol und hydrierten natürlichen Lecitinen (vorzugsweise hydriertem Ei-Lecitin, hydriertem Soja-Lecitin und deren Mischungen) sind und eine Jodzahl von nicht größer als 2 g/100 g aufweisen.

4. Pharmazeutische Zubereitung nach einem der Ansprüche 1, 2 oder 3, wobei die verwendeten enterischen Wirkstoffe im Bereich der Pharmazie zur Herstellung enterischer Beschichtungen für Kapseln und Tabletten üblich und vorzugsweise Versiegelungen sind, die auf Acrylharzen (Acrylsäurecopolymere mit Acrylsäureestern) wie Eudragit L™, Eudragit S™, Eudragit RL™ oder Eudragit RS™ basieren, und Versiegelungen sind, die auf Zellulosederivaten wie Zelluloseaceto-phtalat basieren.

5. Pharmazeutische Zubereitung nach einem der Ansprüche 1, 2, 3 und 4, wobei die zum Erhalt der Liposome verwendeten Lipide eine Mischung aus Cholesterol und hydrierten natürlichen Lecitinen umfassen, dessen Cholesterolanteil zwischen 0 und 75% und insbesondere zwischen 24% und 51% liegt.

## Revendications

1. Préparation pharmaceutique destinée à une administration par voie orale qui comprend des capsules, des comprimés ou des dragées enrobés d'une substance entérique contenant une poudre de liposomes lyophilisée ou évaporée, qui n'est pas mise en suspension dans l'huile, incorporant des diphosphonates comme l'acide alendronique et ses sels.

2. Préparation pharmaceutique destinée à une administration par voie orale, qui comprend des capsules, des comprimés, des dragées enrobés d'une substance entérique contenant une poudre de liposomes lyophilisée ou évaporée, qui n'est pas mise en suspension dans l'huile, incorporant des sels minéraux de fer (II) comme le sulfate de fer (II).

3. Préparation pharmaceutique selon l'une quelconque des revendications 1 et 2, dans laquelle les lipides utilisés pour obtenir ces liposomes comprennent un mélange de cholestérol et de lécithines naturelles hydrogénées (de préférence de la lécithine d'oeuf hydrogénée et de la lécithine de soja hydrogénée et leurs mélanges) ayant un indice d'iode qui n'est pas supérieur à 2 g/100 g.

4. Préparation pharmaceutique selon l'une quelconque des revendications 1, 2 et 3, dans laquelle les agents entériques utilisés sont ceux qui sont normalement utilisés dans le domaine pharmaceutique pour produire des enrobages entériques pour des capsules et des comprimés, de préférence des enrobages étanches à base de résines acryliques (copolymères d'acide acrylique avec des esters d'acide acrylique) comme Eudragit L™, Eudragit S™, Eudragit RL™ ou Eudragit RS™, et des enrobages étanches à base de dérivés de cellulose comme l'acétophtalate de cellulose.

5. Préparation pharmaceutique selon l'une quelconque des revendications 1, 2, 3 et 4, dans laquelle les lipides utilisés pour obtenir ces liposomes comprennent un mélange de cholestérol et de lécithines naturelles hydrogénées dans lequel la proportion de cholestérol varie entre 0 et 75 % et de manière plus spécifique, entre 24 % et 51 %.
